# EUROPÄISCHE PATENTSCHRIFT

(11) **EP 0 867 196 B1**
(45) Veröffentlichungstag und Bekanntmachung des Hinweises auf die Patenterteilung: **20.08.2003**
(21) Anmeldenummer: 98810213.3
(22) Anmeldetag: 13.03.1998
(51) Int. Cl.: A61M 39/02

(54) **Kathetersystem für Hautdurchlassvorrichtungen**
Catheter system for transdermal passages
Système de cathéter pour des passages transdermaux

(30) Priorität: 26.03.1997 CH 72797
(43) Veröffentlichungstag der Anmeldung: 30.09.1998
(73) Patentinhaber: Disetronic Licensing AG, 3401 Burgdorf (CH)
(72) Erfinder: Bestetti, G.E., Prof. Dr., 3098 Köniz (CH); Frei, Thomas, 3432 Lützelflüh (CH); Reinmann, Andreas, 3014 Bern (CH)

(56) Entgegenhaltungen:
- US-A- 4 488 877
- US-A- 4 781 693
- US-A- 5 171 216

## Beschreibung

Die Erfindung bezieht sich auf ein Kathetersystem für Hautdurchlassvorrichtungen gemäss dem Oberbegriff des Anspruchs 1.

Bekannt sind ein- oder mehrlumige Katheter aus Kunststoff die im Extrusionsverfahren hergestellt und auf eine standardisierte Länge zugeschnitten werden. Die Katheterspitze, welche am Ausschüttungsort des Arzneimittels im menschlichen oder tierischen Körper angeordnet wird, wird derart abgerundet, dass die Gefahr einer Verletzung der Gefässe oder der Darmhaut im Bauchraum möglichst vermieden werden kann. Der Katheter wird vom Hersteller absichtlich in einer Länge hergestellt, welche nicht benötigt wird, damit der Katheter während einer Operation durch Wegschneiden des überflüssigen Teils auf die gewünschte Länge gekürzt werden kann. Das durch das Wegschneiden entstehende Katheterende wird nachträglich trichterförmig bearbeitet, so dass eine Koppelung im inneren einer Portkammer mit einem Infusionsset möglich ist.

Als nächstliegender Stand der Technik gilt die US 4,488,877. Sie beschreibt ein Konnektierungssystem, bestehend aus einer Verschlusshülse, einer in der Verschlusshülse angeordneten elastischen Dichtung und einem inneren Katheter, welche untrennbar miteinander verbunden sind.

Aus den Patentschriften US-A-5 306 255 und EP-B-0 302 076 sind sub- und percutane Portkörper bekannt. Diese Portkörper dienen der Verbindung eines ausserhalb des menschlichen oder tierischen Körpers gelegenen Infusionsschlauches, mit einem im inneren des menschlichen oder tierischen Körpers gelegenen Portkatheters. Äussere Formen solcher Portkörper werden in den erwähnten Patentschriften genügend umschrieben, weshalb an dieser Stelle auf diese Schriften verwiesen wird.

Aus der Patentschrift EP-B-0 398 950 ist die innere Ausgestaltung eines Portkörpers, der Portkammer, bekannt. Die Portkammer besteht aus einer Membrankammer, mit zwei gegenüberliegenden Öffnungen, einer zwischen den Öffnungen angeordneten, aus einem elastischen Material bestehenden Membran, welche im Zentrum einen Verbindungskanal zwischen den beiden Öffnungen der Kammer aufweist. Die eine Öffnung dient zur Aufnahme eines externen Infusionsschlauches, während an der anderen ein Portkatheter angeordnet wird.

Bei den bekannten Vorrichtungen wird der Portkatheter derart mit der Portkammer verbunden, dass nachdem der Katheter am einen Ende trichterförmig bearbeitet wurde, die Portkatheterspitze mit dem abgerundeten Ende durch den Port geschoben wird, bis sich der trichterförmige Teil des Portkatheters in der dafür vorgesehene Öffnung des Portkörpers verkeilt.

Nachteilig an den bekannten Systemen ist, dass für die mechanische Nachbearbeitung des Katheterendes, welche diesem die erwähnte Trichterform gibt, eine Spezialvorrichtung notwendig ist, deren Bedienung geschult werden muss. Die Vorrichtung muss in jedem Spital vorhanden sein, wodurch Kosten für Anschaffung, Wartung, Reinigung und Sterilisation entstehen. Die Nachbearbeitung des Katheterendes nimmt auch eine gewisse Zeit während der Operation in Anspruch. Im weiteren kann im Operationssaal eine Kontrolle der Funktionsfähigkeit nach der Veränderung des Katheterendes nur optisch erfolgen.

Nach der Bearbeitung muss, wie oben erwähnt, das Katheterende in seine Endposition durch den Portkörper geschoben werden. Dazu ist eine Krafteinwirkung direkt am zuvor bearbeiteten Katheterende notwendig. Hierbei besteht die Gefahr, dass das Katheterende beschädigt, verunreinigt oder unsachgemäss montiert wird, so dass der Arzneimittelkanal verstopft wird. Ein weiterer Nachteil besteht darin, dass der Katheter erst im eingebauten Zustand auf seine Funktionsfähigkeit geprüft werden kann.

Hier will die Erfindung Abhilfe verschaffen. Der Erfindung liegt die Aufgabe zugrunde, ein Kathetersystem für die Verabreichung von Arzneimittel zu entwickeln, das weder eine mechanische Nachbearbeitung eines Katheterendes vom Operateur verlangt, noch zulässt, einfach und sicher in der Handhabung ist und schnell implantiert werden kann. Das Kathetersystem muss auch ausserhalb des Portes und vor der Implantation auf seine Funktion getestet werden können.

Die Erfindung löst die gestellte Aufgabe mit einem Kathetersystem, welches die Merkmale des Anspruchs 1 aufweist.

Die durch die Erfindung erreichten Vorteile sind im wesentlichen darin zu sehen, dass während der Operation weder eine mechanische Nachbearbeitung des Katheters vom Operateur verlangt wird, noch möglich ist, und eine einfache, sichere und rasche Handhabung der Fixierung des Kathetersystems im Portkörper möglich ist.

Ein bevorzugtes Beispiel der Erfindung ist in den Figuren dargestellt.

Es zeigen:
- Fig. 1: Einen Portkörper
- Fig. 2: Ein erfindungsgemässes Kathetersystem

Im folgenden bedeuten die Begriffe innen = innerhalb des menschlichen oder tierischen Körpers und aussen = ausserhalb des menschlichen oder tierischen Körpers.

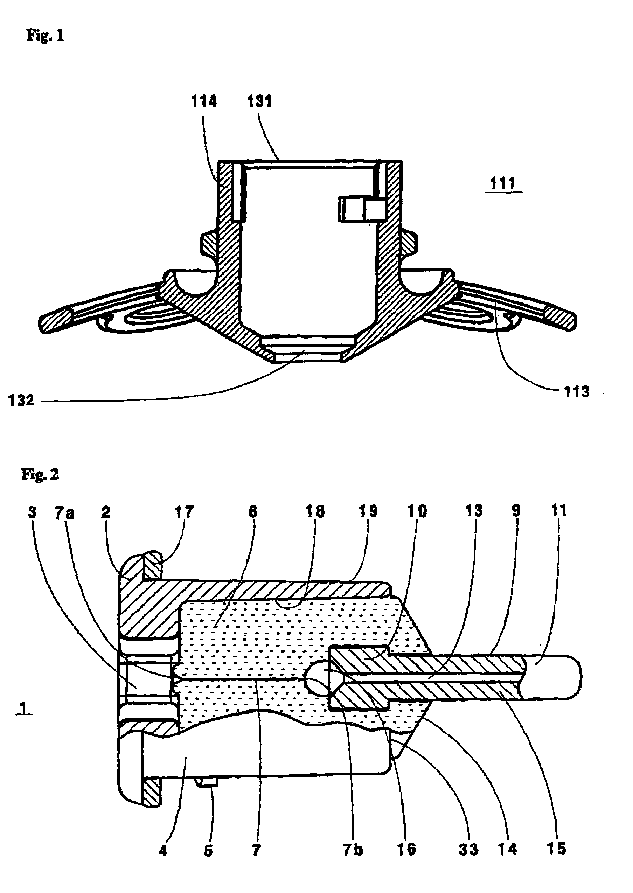

Wie in den Fig. 1 und 2 dargestellt, wird eine Verschlusshülse 4 eines Kathetersystems 1 in einen Portkörper 111 eingesetzt. Ein Portkörper 111 besteht aus einer hohlzylindrischen Hülse 114 und einem daran angeordneten radialen Verankerungsteller 113. Der Portkörper 111 weist zwei gegenüberliegende Öffnungen 131,132 auf. Eine gegen aussen liegende Öffnung 131 entspricht dem Innendurchmesser der hohlzylindrischen Hülse 114. Die zweite Öffnung 132 befindet sich am Ende des kegelförmig auslaufenden Portkörpers 111.

Wie aus Fig. 2 ersichtlich, besteht das Kathetersystem 1 , aus einer hohlzylindrischen Verschlusshülse 4, einer teilweise in der Verschlusshülse 4 angeordneten elastischen Dichtung 8 und einem schlauchförmigen Katheter 9.

Der Katheter 9 wird vorzugsweise aus einem biokompatiblen Kunststoff hergestellt und kann in drei Bereiche eingeteilt werden: einen Katheterkopf 10, einen schlauchförmigen Zwischenbereich 15 und einen abgerundeten Endbereich 11. Im inneren des Katheters 9 verläuft ein durchgehender Arzneimittelkanal 13.

Der Aussendurchmesser des Katheterkopfes 10 ist grösser, als derjenige des schlauchförmigen Zwischenbereiches 15. Der Übergang zwischen Katheterkopf 10 und Zwischenbereich 15 ist vorzugsweise als Stufe ausgestaltet.

Der innere Teil des Katheterkopfes 10 verläuft trichterförmig in den Arzneimittelkanal 13.

Die Verschlusshülse 4 ist zylindrisch aufgebaut und weist zwei gegenüberliegende Öffnungen 3,33 auf. Die gegen aussen liegende Öffnung 3 befindet sich im Zentrum eines Deckels 2, welcher die Verschlusshülse 4 gegen aussen abdeckt. Die zweite, gegen innen liegende Öffnung 33 entspricht dem Innendurchmesser der Verschlusshülse 4.

Die elastische Dichtung 8 füllt die Verschlusshülse 4 aus und überlappt die Öffnung 33 derart, dass sie die Verschlusshülse 4 kegelförmig 14 abschliesst. Die elastische Dichtung wird vorzugsweise aus einem elastischen Kunststoff wie Silikon oder Polyurethan hergestellt. Im Zentrum der elastischen Dichtung ist ein selbstschliessender Verbindungskanal 7 angeordnet. Eine gegen aussen liegende Einlassöffnung 7a des Verbindungskanals 7 befindet sich bei der Öffnung 3 des Deckels 2, während sich eine Auslassöffnuag 7b des Verbindungskanals 7 im Inneren der elastischen Dichtung befindet. Bei der Auslassöffnung 7b wird der Katheterkopf 10 angeordnet, so dass Verschlusshülse 4, elastische Dichtung8 und Katheter 9 ineinander übergehen.

Die Verschlusshülse 4 umschliesst die elastische Dichtung8 und den darin angeordneten Katheterkopf 10 derart, dass die elastische Dichtung 8 und der Katheterkopf 10 zentrisch unter einem definierten Vorspanndruck zwischen dem Deckel 2 und der Verschlusshülseninnenwand 18 zusammengehalten werden.

Der Aussenmantel 19 der Verschlusshülse 4 ist mit bis zu drei Verschlussnocken 5 versehen, so dass die Verschlusshülse 4 und damit das gesamte Kathetersystem 1 in einem entsprechend ausgestalteten zylindrischen Portgehäuse 114 montiert werden kann. Der Durchmesser des Deckels 2 der Verschlusshülse 4 entspricht dem Aussendurchmesser des Portgehäuses 114. Eine zwischen dem Deckel 2 und dem Portgehäuse 114 angebrachte Verschlussdichtung 17 sorgt dafür, dass kein Schmutz und keine Bakterien zwischen das Portgehäuse 114 und die Verschlusshülse 4 gelangen können.

Die Verschlusshülse 4 wird vorzugsweise aus Kunststoff im Spritzgussverfahren hergestellt. Dadurch ist es möglich die Verschlusshülse 4 oder zumindest den Deckel 2 bereits bei der Herstellung hautähnlich zu färben, so dass der von aussen sichtbare Deckel 2 optisch unauffällig ist.

Der Bedarf nach einer optimalen Katheterlänge wird dadurch erfüllt, dass der Katheter 9 fabrikationsmässig in unterschiedlichen Längen hergestellt wird.

Das erfindungsgemässe Kathetersystem 1 wird derart in ein Portgehäuse 114 eingesetzt, dass zuerst der Katheter 9, durch die entsprechende Öffnung 132 des Portgehäuses 114 geschoben wird. Sobald der kegelförmige Bereich 14 der Membran 8 an die Innenwand des Portgehäuses 114 anschlägt, werden mittels einer leichten Drehung der Verschlusshülse 4 die am Aussenmantel 19 der Verschlusshülse 4 angeordneten Verschlussnocken 5 in einer entsprechenden Aussparung des Innenmantels des Portgehäuses 114 verankert. Die leichte Drehung der Verschlusshülse 4 wird dadurch ermöglicht, dass die Deckelöffnung 3 als Sechskant geformt ist. Selbstverständlich sind auch andere Schlüsselformen denkbar.

Um Arzneimittel von einem Behälter in einen menschlichen oder tierischen Körper abzugeben, wird von aussen ein Infusionsschlauch durch die Öffnung 3 des Deckels 2 und durch den Verbindungskanal 7 zum Katheterkopf 10 geführt. Um zu verhindern, dass der Infusionsschlauch sich aus dieser Verbindung allzu leicht löst, kann das Ende des Infusionsschlauches derart ausgeführt werden, dass es in einer entsprechenden Aussparung 16 zwischen elastischer Dichtung 8 und Katheterkopf 10 festgehalten wird.

wird der Infusionsschlauch entfernt, kann auf die Öffnung 3 ein Deckel aufgesetzt werden.

## Patentansprüche

1. Kathetersystem (1) für eine Hautdurchlassvorrichtung (111), bestehend aus einer Verschlusshülse(4), einem Katheter (9) und einer teilweise in der Verschlusshülse (4) angeordneten elastischen Dichtung (8), derart in einer Hautdurchlassvorrichtung (111) befestigbar, dass der Katheter (9) aus der Hautdurchlassvorrichtung (111) gegen das Körperinnere herausragt und in der elastischen Dichtung (8) gehalten wird, wobei die einzelnen Bestandteile des Kathetersystems (1) untrennbar miteinander verbunden sind, und wobei die in der Verschlusshülse (4) angeordnete elastische Dichtung (8) einen selbstschliessenden Verbindungskanal (7) aufweist,
**dadurch gekennzeichnet, dass** an der Hülsenaussenwand (19) Verschlusselemente (5) angebracht sind, um in entsprechenden Gegenelementen der Hautdurchlassvorrichtung lösbar montiert zu werden.

2. Kathetersystem nach Anspruch 1, **dadurch gekennzeichnet, dass** die Verschlusshülse (4) auf der einen Seite von einem Deckel (2), in dessen Zentrum sich eine Öffnung (3) befindet, abgeschlossen wird.

3. Kathetersystem nach Anspruch 2, **dadurch gekennzeichnet, dass** die elastische Dichtung (8) die Verschlusshülse (4) ausfüllt und auf der, dem Deckel gegenüberliegenden Seite (33), kegelförmig abschliesst.

4. Kathetersystem nach Anspruch 1 **dadurch gekennzeichnet, dass** der in der elastischen Dichtung (8) angeordnete verbindungskanal (7) die Öffnung (3) im Deckel (2) mit dem Katheter (9) verbindet.

5. Kathetersystem gemäss einem der Ansprüche 1 bis 4, **dadurch gekennzeichnet, dass** die Verschlusselemente (5) in den entsprechenden Gegenelementen der Hautdurchlassvorrichtung (111) lösbar eingerestet werden können.

6. Kathetersystem gemäss einem der Ansprüche 1 bis 5, **dadurch gekennzeichnet, dass** es eine hautähnliche Farbe hat.

7. Kathetersystem nach einem der Ansprüche 2 bis 6, **dadurch gekennzeichnet, dass** die Öffnung (3) des Deckels (2) als Innensechskant ausgebildet ist.

8. Kathetersystem nach einem der Ansprüche 1 bis 6, **dadurch gekennzeichnet, dass** das Kathetersystem (1) aus einem organischen Material, vorzugsweise Kunststoff, hergestellt wird.

## Claims

1. A catheter system (1) for a transdermal passage device (111), comprising a closure sleeve (4), a catheter (9) and an elastic seal (8) arranged partly in the closure sleeve (4), fixable in a transdermal passage device (111) in such a way that the catheter (9) projects towards the inside of the body out of the transdermal passage device (111) and is held in the elastic seal (8), wherein the individual components of the catheter system (1) are inseparably connected together, and wherein the elastic seal (8) arranged in the closure sleeve (4) has a self-closing communicating passage (7), **characterised in that** closure elements (5) are mounted to the outside wall (19) of the sleeve in order to be releasably fitted in corresponding counterpart elements of the transdermal passage device.

2. A catheter system according to claim 1 **characterised in that** the closure sleeve (4) is closed on the one side by a cover (2), in the centre of which there is an opening (3).

3. A catheter system according to claim 2 **characterised in that** the elastic seal (8) fills up the closure sleeve (4) and closes it in a tapered configuration on the side (33) opposite to the cover.

4. A catheter system according to claim 1 **characterised in that** the communicating passage (7) in the elastic seal (8) communicates the opening (3) in the cover (2) with the catheter (9).

5. A catheter system according to one of claims 1 to 4 **characterised in that** the closure elements (5) can be releasably latched in the corresponding counterpart elements of the transdermal passage device (111).

6. A catheter system according to one of claims 1 to 5 **characterised in that** it is of a skin-like colour.

7. A catheter system according to one of claims 2 to 6 **characterised in that** the opening (3) in the cover (2) is in the form of a hexagonal recess.

8. A catheter system according to one of claims 1 to 6 **characterised in that** the catheter system (1) is made from an organic material, preferably plastic material.

## Revendications

1. Système de cathéter (1) pour un dispositif de passage transdermique (111), comprenant une douille de fermeture (4), un cathéter (9) et un joint élastique (8) agencé partiellement dans la douille de fermeture (4), susceptible d'être fixé dans un dispositif de passage transdermique (111) de sorte que le cathéter (9) dépasse hors du dispositif de passage transdermique (111) vers l'intérieur du corps et est maintenu dans le joint élastique (8), les composants individuels du système de cathéter (1) étant inséparablement reliés entre eux et le joint élastique (8) agencé dans la douille de fermeture (4) présentant un canal de liaison auto-fermant (7), **caractérisé en ce que** des éléments de fermeture (5) sont placés sur la paroi extérieure de la douille (19) pour être montés de manière amovible dans des éléments antagonistes correspondants du dispositif de passage transdermique.

2. Système de cathéter selon la revendication 1, **caractérisé en ce que** la douille de fermeture (4) est fermée sur un côté par un couvercle (2) au centre duquel se trouve un orifice (3).

3. Système de cathéter selon la revendication 2, **caractérisé en ce que** le joint élastique (8) remplit la douille de fermeture (4) et forme une fermeture conique sur le côté (33) opposé au couvercle.

4. Système de cathéter selon la revendication 1, **caractérisé en ce que** le canal de liaison (7) agencé dans le joint élastique (8) relie l'ouverture (3) dans le couvercle (2) au cathéter (9).

5. Système de cathéter selon l'une des revendications 1 à 4, **caractérisé en ce que** les éléments de fermeture (5) peuvent être enclenchés de manière amovible dans les éléments antagonistes correspondants du dispositif de passage transdermique (111).

6. Système de cathéter selon l'une des revendications 1 à 5, **caractérisé en ce qu'**il est de couleur semblable à la peau.

7. Système de cathéter selon l'une des revendications 2 à 6, **caractérisé en ce que** l'orifice (3) du couvercle (2) est de forme hexagonale.

8. Système de cathéter selon l'une des revendications 1 à 6, **caractérisé en ce que** le système de cathéter (1) est fabriqué à partir d'un matériau organique, de préférence de la matière synthétique.
